# EUROPEAN PATENT APPLICATION

(11) **EP 2 417 908 A1**
(43) Date of publication of application: **15.02.2012**
(21) Application number: 10172605.7
(22) Date of filing: 12.08.2010
(51) Int. Cl.: A61B 5/113, G01S 13/56

(54) **Device, system and method for measuring vital signs**

(71) Applicant: Philips Intellectual Property & Standards GmbH, 20099 Hamburg (DE); Koninklijke Philips Electronics N.V., 5621 BA Eindhoven (NL)
(72) Inventor: The designation of the inventor has not yet been filed
(74) Representative: Golla-Franz, Anke Lucia

(57) **Abstract**

In order to provide means for measuring vital signs such as heart rate, breathing rate, pulse forms, that allow accurate and unobtrusive signal acquisition and that are automatically adjusted to varying positions of an area of interest within a predetermined area, a device, a system and a method for measuring vital signs are provided, wherein electromagnetic signals such as microwave or radar are emitted and reflected electromagnetic signals are sensed with directional sensitivity by a transceiving unit and wherein a direction of interest is determined based on the sensed reflected signals and a primary direction of sensitivity (300) of the transceiving unit is adjusted to the direction of interest by a control unit. Thus, vital signs can be monitored in an unobtrusive and simple way.

## Description

The present invention relates to a device, a system and a method for measuring vital signs, wherein a direction of sensitivity is automatically adjusted.

### BACKGROUND OF THE INVENTION

For many home applications as well as in personal health care and patient monitoring, measuring vital signs of a person is believed to be essential for sustainable future health care systems, because this will help to sufficiently lower health care costs. Conceivable scenarios are not only the supervision of patients at home, but also the detection of slowly developing diseases, i.e. long-term monitoring of (still relatively) healthy people for the purpose of prevention. In this home care or personal health area, unobtrusiveness of the vital sign measurements is of paramount importance for the measurement to be accepted by the user. The same holds for consumer lifestyle applications related to relaxation and sleep, , when the state of the user should be monitored comfortably with a sensor solution in, e.g. a alarm clock, wake-up light- or sleep and relaxation products (sleep paced breathing). In paced breathing, for instance, persons get distracted by focusing on an actuator that helps them to breathe slowly, get relaxed and fall asleep easily. For such an exercise to be very effective, it is advantageous to measure an actual breathing state of the user, with as little interference as possible. Therefore, solutions without any physical body contact are always highly preferred.

It has been shown, that it is possible to measure vital signs by means of electromagnetic waves or radar from a distance. For instance, chest motion due to respiratory action as well as physical activity can be detected. However, measuring vital signs of a person from a distance with the help of electromagnetic waves requires that the waves are thoroughly directed towards the person and for many applications even towards a particular body part of that person. Thus, when measuring vital signs of a person by means of electromagnetic radiation, the positioning of the transceiving device is of uttermost importance. When the device is positioned, e.g. on a bedside table for measuring the breathing state of a target person, several problems occur with respect to the proper orientation of the device towards the area of interest of the target person (i.e. the chest area of the person). For instance, when the target person lying on his back has his arm next to him in front of the transceiving device, the breathing of this person cannot be properly measured. Moreover, in a double bed with two persons, a second person may be measured as well, resulting in meaningless data.

Another difficulty is that the transceiving device must still remain directed towards a target person, though the person may move. Even if radar is used to monitor the respiration of a person in bed, the person can still turn around and move out of the limited area of sensitivity of the radar. The area of sensitivity of a directional radar transceiving unit is determined by the antenna design and comprises typically a main lobe, i.e. a direction of highest sensitivity, and a few small side lobes. Thus, the area or direction of sensitivity of the transceiving device is limited, while the location of the user is likely to vary in bed, the area of interest (e.g. the chest area of the user) moving with him. In common vital sign monitoring systems, this requires therefore that the transceiving device is carefully arranged and that the user remains motionless in the area of sensitivity.

### SUMMARY OF THE INVENTION

It is therefore an object of the present invention to provide a device, a system and a method for measuring vital signs that allow accurate and unobtrusive signal acquisition and that are automatically adjustable to varying positions of the area of interest within a predetermined area.

The object is achieved by the features of the independent claims. The idea of the present invention relies on determining an area of interest, i.e. a direction of the area of interest, and subsequently adjusting a primary direction of sensitivity, i.e. a direction of highest sensitivity, to the determined direction of interest in order to measure vital signs in this direction. Thus, a device for measuring vital signs comprises a transceiving unit for emitting and receiving electromagnetic signals. Depending on the embodiment of the device, the direction of sensitivity may be determined by the emission and receiving direction or by the receiving direction only. The device comprises further a control unit for determining a direction of interest based on the sensed reflected signals, so that a primary direction of sensitivity of the transceiving unit can be adjusted to this direction of interest. By these means, although the area of sensitivity of the device is limited, the area of sensitivity can be automatically adjusted to a varying direction of interest. For instance, if the device is used for measuring breathing parameters of a person in bed, a primary direction of sensitivity of the device can automatically follow the moving person. In another application, the device may be attached to or incorporated in a driver's and/or passenger's seat or in the steering area of a vehicle, train or boat for drowsiness detection, awareness monitoring and/or health state monitoring. Likewise, the device may be applied in an airplane, e.g. in a pilot's or passenger's seat.

Preferably, the wavelengths of the electromagnetic signals are in the range of radio waves or radar signals, since radiation at these wavelengths is not harmful to health.

In a preferred embodiment, directional sensing is performed by scanning an emission direction of a main beam (main lobe) of electromagnetic signals over a predetermined range of angles. Alternatively or additionally, also the primary receiving direction may be scanned over a predetermined range of angles. Thus, in order to sense electromagnetic signals reflected back from the area of interest to the transceiving unit with directional sensitivity, the transceiving unit may have a plurality of distinguishable emission directions and/or receiving directions in order to provide a plurality of directions of sensitivity. In general, the transceiving unit may comprise one or more transceivers, receivers and/or transmitters. Preferably, at least one of the transceivers, transmitters and receivers of the transceiving unit is a directional antenna. By directing the emitting and/or receiving direction of the device to a determined direction of interest, it is not necessary to provide duplicate sensors to completely cover a predetermined area, in which the area of interest can be located, e.g. the area of a bed or a room. Hence, production costs can be saved. Furthermore, the user must not be instructed to aim the device carefully or to keep his body inside a limited area, thus increasing the user convenience.

In another embodiment of the present invention, the transceiving unit comprises a plurality of directional receiver antennas and one emitting antenna capable of emitting to the whole predetermined area, in which the area of interest can be located. In this embodiment, a switching process is simplified by not switching an array of transceiver antennas, but splitting in transmitting and receiving antennas. Thus, only the receiver antennas have to be switched.

Preferably, the transceiving unit comprises a plurality of directional antennas, such as at least one of a phased antenna array, a Yogi antenna, a parabolic antenna, a logarithmic-periodic antenna, a corner-reflector antenna and a patch antenna. In one preferred embodiment, beam forming is performed with a cylindrically or spherically arranged antenna array, so that a room can be scanned in a full 360° range. Thereby, the antenna elements may be segmented in a vertical direction. If the antenna elements are also segmented in a horizontal direction, the shape of an emitted beam and thus the direction of sensitivity can be formed arbitrarily.

In another embodiment, the transceiving unit comprises at least two transceivers, wherein the transceiver with the best reflected signal can be chosen or wherein the signals from all transceivers can be combined to enhance the received signal. Preferably, the transceiving unit comprises at least two transceivers that are shifted by a quarter wavelength in height or by 180° in phase. Due to the radar path being constituted by the trajectory towards and from the target, the displacement also results in a shift of 180° in phase. This facilitates the extraction of real time vital signals and the detection of small signal amplitudes (i.e. when the excursion of the body is very small). Possibly, also antennas having different wavelengths are included in the transceiving unit.

It is preferred that the device is configured such that it can be positioned close to a body. For this, the device may be designed especially flat or small, so that it can be easily placed under, integrated in or attached to a mattress or chair. Due to the proximity of the sensing device to the body, better signal noise ratio can be achieved, while using electromagnetic radiation of lower energy. Moreover, the signals are unambiguously and reliable coming from a person of interest and cannot be confused with signals coming from a person close to the person of interest. In addition, the device can be installed unobtrusively so that it is not visible or noticeable to a user.

Preferably, the direction of interest is determined based on a signal-to-noise ratio of the received reflected signals. Alternatively, any other means for estimating the signal quality may be used. In general, the direction of interest may relate to the direction of the largest signal amplitude. For instance, when measuring the breathing state of a user, the direction of interest relates to the direction of the chest of the user, from which reflected signals having the largest amplitude can be expected.

In a preferred embodiment, the direction of interest is determined in periodic intervals, at predetermined points in time, e.g. every minute, and/or each time when a predetermined condition is fulfilled. For instance, the direction of interest may be updated when the device is switched on, when an input signal is received, when the presence of a person is detected, when the reflected signal has changed significantly, when no vital signs can be extracted from the sensed reflected signals and/or when it has been determined that the reflected signal has been lost. Preferably, the device first polls and scans a small angle around the previous direction of interest in order to find the new best direction having the strongest signal. This is particularly suitable for small variations of the new direction of interest from the previous direction of interest, i.e. small or slow movements of the person, since the device does not have to scan the whole range of possible directions of sensitivity. Thus, the correction of the direction of interest is accelerated and simplified.

Preferably, after the direction of interest has been determined, the device can measure vital signs by sensing the reflected electromagnetic signals in the primary direction of sensitivity for a predetermined time.

In a further embodiment, the device can be operated in a stand-by mode, wherein the device is activated in predetermined time intervals in order to determine whether a person is present. If a person is present, the device is fully activated for measuring vital signs. For this, it may be determined that a person is present either when detecting general motion or vital signs by the device. If no person is present, the device returns into the resting state of the stand-by mode and repeats the presence detection after the predetermined time interval. By these means, the device can be automatically switched on and off, thus reducing energy consumption.

The device may further comprise a modulation unit for modulating signals, e.g. by means of frequency modulation, amplitude modulation, pulse modulation etc. This may be useful, if more than one device is used in close proximity to each other.

Furthermore, the device may include an input unit for receiving input by the user, e.g. settings, parameters, time intervals, processing parameters, on/off signals, and the like. The input unit may either be provided with the device or may be in wired and/or wireless communication with the device, i.e. employed as a remote control.

Possibly, the device comprises further an output unit for visually and/or acoustically providing feedback, information and/or instructions. This is particularly required, if the device is used for paced breathing, yoga or breathing training.

In addition may comprise a communication unit for wired and/or wireless data transmission to external devices, such as computers, PDAs, mobile terminals etc.. In order to evaluate a time history, storage means may also be provided in order to store recorded vital signs, received reflected signals, settings and/or parameters, etc.

In a further aspect of the present invention, a system for measuring vital signs is provided. The system provides a transceiving unit that can emit electromagnetic signals to a predetermined area, in which the area of interest is expected to be located. The transceiving unit is further able to receive reflected electromagnetic signals with directional sensitivity, e.g. the transceiving unit is able to distinguish the directions, from which the reflected electro-magnetic signals are received. In addition, the system comprises a control unit for determining a direction of interest based on the sensed reflected signals. For instance, when the breathing state of a person is monitored, the direction of interest corresponds to the direction of the person's chest with respect to the transceiving unit. This direction may be determined by considering predetermined signal characteristics, e.g. the signal amplitude, the signal-to-noise ratio or the like. The control unit can further adjust a primary direction of sensitivity of the receiving unit to the direction of interest. By these means, a large predetermined area can be covered, in which the area of interest may be located, without providing a multitude of transceiving units or restricting the person to stay in a certain area. Since the transceiving unit and the control unit may be provided separately from each other, they are adapted to be interconnected for wired and/or wireless communication. Thus, the transceiving unit and the control unit can exchange data in one or in both directions. This embodiment has the advantage, that the transceiving unit can be configured to be particularly flat and small, when the control unit is provided separately. Also, when using a computer or the like as a control unit, the control unit already comprises storage and input means. Thus, the amount of hardware required for the system can be further reduced.

In a further aspect of the present invention, a method for measuring vital signs is provided. In this method, first, electromagnetic signals are emitted to a predetermined area by means of a transceiving unit. Preferably, this is performed, when the transceiving unit is switched on. The predetermined area may relate to an area, in which the area to be measured can be expected. In a next step, the transceiving unit that is directionally sensitive receives reflected electromagnetic signals. Based on these reflected signals, a direction of interest is determined and a primary direction of sensitivity of the transceiving unit is adjusted to the direction of interest. Preferably, after having adjusted the primary direction of sensitivity of the transceiving unit, reflected electromagnetic signals are measured for a predetermined time and vital signals are derived from these detected signals.

The method may further comprise a step of detecting a presence of a person, e.g. by motion detection or by detection of vital signs. If it is determined that a person is present, the above described method for measuring vital signs is started. If no person is present, a stand-by mode is activated, in which presence detection is repeated in predetermined time intervals. Thus, by only activating the method for measuring vital signs, when a person is present, energy can be saved by automatically switching on and off corresponding devices.

Preferably, in the method for measuring vital signs, different phases of a periodic vital sign are determined based on the shape of the reflected signals. For instance, exhaling and inhaling may be determined based on characteristic signal shape.

### BRIEF DESCRIPTION OF THE FIGURES

- Fig. 1: illustrates a possible application for a device according to the present invention.
- Fig. 2: illustrates another arrangement of a device according to the present invention, having two transceivers.
- Fig. 3: illustrates an embodiment for a radar sub-system.
- Fig. 4: shows an example for a received signal when monitoring a breathing state of a person.
- Fig. 5: illustrates schematically an angular dependence of the detection of vital signs.
- Fig. 6A and 6B: illustrate other embodiments for a radar sub-system according to the present invention, having a plurality of transceiver antennas and a plurality of receiver antennas as well as one fixed transmitter antenna, respectively.
- Fig. 7A: illustrates the directional characteristic of a simulated radiation pattern of a four-element phased patch antenna array and Fig. 7B illustrates schematically beam forming in a phased antenna array.
- Fig. 8: illustrates an exemplary arrangement of transceivers for beam forming.
- Fig. 9A: illustrates a flow diagram of a method according to the present invention;
- Fig. 9B: illustrates a method for measuring vital signs according to an embodiment of the present invention.
- Fig. 10: illustrates signal recording and processing according to the present invention.
- Fig. 11A and 11B: illustrate two alternatives of signal processing.
- Fig. 12: illustrates signal processing, when the device is switched to another receiving direction.

### DETAILED DESCRIPTION

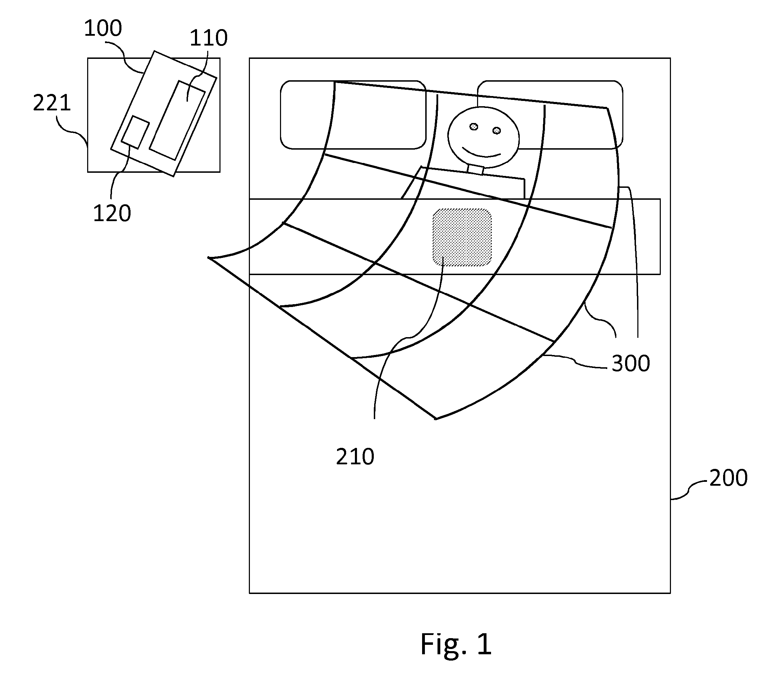

In Fig. 1, an exemplary embodiment of a device 100 according to the present invention is shown. A device 100 for measuring vital signs, such as heart rate, breathing rate or pulse forms, comprises at least a transceiving unit 110 for emitting and receiving electromagnetic waves and a control unit 120 for controlling the transceiving unit 110. The control unit 120 can process the received signals in order to determine a target direction, from which the strongest or best signal is received. Then, a measurement direction or direction of sensitivity 300 (sensitivity direction), in which measurements of vital signs are performed, can be adjusted to this target direction. As will be described below, the adjustment of the measurement direction can be performed depending on the embodiment of the device 100 either by switching to a certain antenna, when the device comprises a plurality of antennas 111 each having a fix direction of sensitivity 300, or by swaying the direction of sensitivity 300 of a phased antenna array within the possible range.

When monitoring the breathing state of a sleeping person, the device 100 may be positioned on a bedside table 221 next to a bed 200, in which the user is sleeping. In order to determine vital signs, a beam of electromagnetic waves is emitted by the device 100 to an area of interest 210 on the user's body. Depending on the application and the kind of vital signs to be monitored, the target area or area of interest 210, towards which the emitted beam is to be directed, can vary. In the example of monitoring the breathing state during sleep, the area of interest 210 corresponds to the chest area of the user. Thus, a transceiving unit 110 of the device 100 aims its direction of sensitivity 300 defined by the emitted beam towards the chest of the user. The emitted radiation is reflected by the chest wall and received by the transceiving unit 110 of the device 100. Due to the respiratory motion, the reflected signal comprises information about the breathing state of the user, e.g. the breathing rate, the momentary phase of breathing (exhaling/inhaling/holding breath), the intensity of breathing, the chest movement or the like. This information may then be used by devices for aiding in relaxation or sleep, e.g. alarm clocks, wake-up lights, sleep-paced-breathing products etc..

In another application for the automotive sector, the device 100 may be integrated in a driver's seat, or in a steering area of a vehicle to monitor the breathing activity, breathing status and heart rate of a subject e.g. for awareness monitoring or to detect critical health states. For this, the transceiving unit 110 comprises an antenna array with a plurality of antennas each having a certain direction of sensitivity. When the device 100 determines a driver being present at the location of the area of interest 210, the antenna is selected that emits in direction of the area of interest 210. Then, signals are recorded in that direction for monitoring vital signs. Preferably, - energy consumption and transmission power is minimized by a deactivation of antennas not being needed. Possibly, the state of health of the driver is monitored with respect to heart functions and breathing. If abnormal vital signs have been obtained, an alarm may be triggered, bystanders can be informed or emergency units are automatically called for help.. Alternatively or additionally, a passenger seat is provided with the device 100 for monitoring a passenger.

In the following description, exemplary embodiments of the present invention are described for monitoring the breathing state of a user during sleep, however, the present invention is not limited to this kind of vital signs. Likewise, a device using radar is described in the following. However, the present invention is not limited thereto, but the device 100 may comprise a transceiving unit 110 for emitting and receiving any kind of electromagnetic radiation.

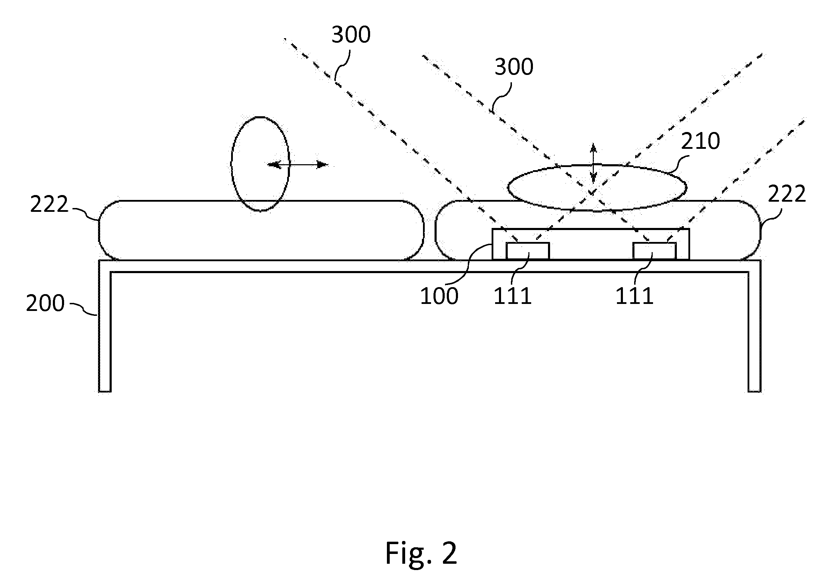

The position of the device 100 with respect to the user is crucial for measuring the correct breathing state. In fact, every object close enough to the device 100 and within its measuring area and thus in its direction of sensitivity 300 will be measured. If the device 100 is positioned on a bedside table, for instance, the breathing state of the target person cannot be properly measured, when the target person is lying on the back and has his arm next to him in front of the device 100. Moreover, in case of a double bed 200 with two persons sleeping, also the second person may unintendedly be measured in addition to the target person. For these reasons, it is preferred to position the device 100 for monitoring vital signs during sleep either underneath a mattress 222 or underneath a bed frame. The most preferred position, though, is underneath the mattress 222 as shown in Fig. 2, since there, the device100 is less noticeable and less obtrusive to the user. Moreover, the signal is not scattered or affected by the bed frame itself. For other applications, e.g. for awareness monitoring in vehicles, trains, boats or airplanes by monitoring breathing state or heart rate, the device 100 may be similarly integrated in a seat or chair. Preferably, the device 100 for measuring vital signs is designed small, flat or compact according to the preferred positioning of the respective application, so that it can be easily installed without being noticed. In general, it is preferred to position the device 100 for monitoring vital signs as close as possible to the area of interest 210 or to the user, so that locating the area of interest 210 and aligning the direction of sensitivity 300 towards it is simplified and the signal-to-noise ratio of the received signals is advantageous.

In order to provide different directions of sensitivity 300, the transceiving unit 110 of the device 100 may comprise a plurality of directional antennas 111 that can be switched. Examples for such directional antennas are Yagi antennas, parabolic antennas, corner-reflector antennas, logarithmic-periodic antennas or patch antennas. The simplest form, however, having still an essentially directional beam is the patch antenna. An array of patch antennas is easily and cheaply manufactured with common printed circuit board technologies. The directional nature may be sharpened by use of a metal cone structure.

Thus, according to one embodiment as shown in Fig. 2, a device 100 having two antennas 111 is positioned under one of the mattresses 222 of a double bed 200, on which two persons are sleeping on the side and on the back, respectively. The arrows indicate the direction of chest movement during breathing. In this embodiment, each antenna 111 has a respective direction of sensitivity 300, so that a more defined and larger measuring area of the device 100 can be achieved. Thus, it becomes less likely that the target person will inadvertently move out of the measuring area. Obviously, due to the selective alignment of the measuring direction, it is very unlikely that the transceiving unit 110 picks up any signals from the second person. As described above, only the antenna emitting towards the area of interest 210 may be operated in order to save energy and reduce the radiation exposure of the user. Therefore, when more than one antenna 111 (or transceiving units 110) are provided, the antennas 111 (or transceiving units 110) are preferably switchable and the antenna 111 (or transceiving unit 110) receiving the best signal can be chosen. In this embodiment, the device may be configured to monitor more than one person by switching between the corresponding directions of sensitivity 300. For this, each person has to stay in a different predetermined area that is then scanned in order to locate the area of interest 210 of the respective person. Alternatively, signals received by all antennas 111 (or transceiving units 110) can be combined to enhance the received signal. In another embodiment, a plurality of antennas 111 (or transceiving units 110) may be used, which are arranged in the cross direction or in the length direction of the bed 200. Moreover, the antennas 111 (or transceiving units 110) may be arranged in different heights, shifted in phase or emitting at different wavelengths. For instance, when using two transceiving units 110, the transceiving units 110 may be shifted by a quarter wavelength of the emitted radiation in height from each other. Since the total trajectory is constituted by the way to and from the reflecting surface, this will result in a phase shift of 180° making it easier to extract the real time breathing signal. This is particularly advantageous, when the excursion of the body is very small. Obviously, the two transceiving units 110 may also directly be shifted in phase by 180° from each other.

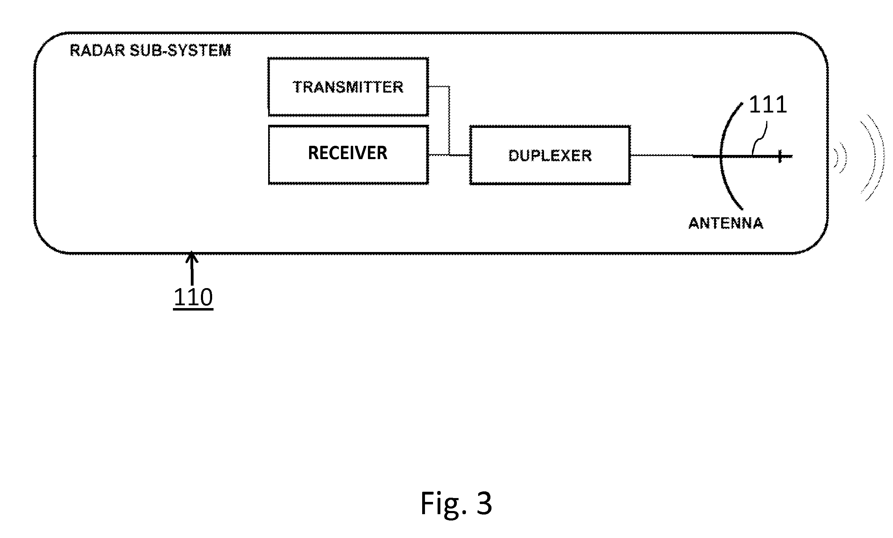

In Fig. 3, an exemplary radar sub-system of a transceiver unit 110 for emitting and receiving radar radiation is shown. The sub-system comprises a receiver and a transmitter both connected via a duplexer to an antenna 111. The radar sub-system may be coherent pulsed, continuous wave or frequency modulated. Possibly, a Doppler radar sub-system is employed in any of the embodiments of the present invention, making use of the Doppler effect to obtain velocity information about moving objects at a distance. For this, after beaming a microwave or radar signal towards the desired target and receiving the reflection, it is analyzed how the frequency of the reflected signals has been altered by the object's motion.

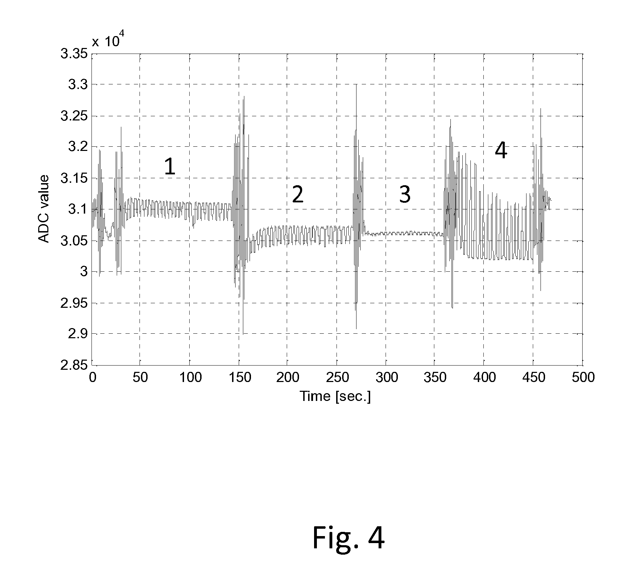

In Fig. 4, an example for received signals is shown, when the device 100 is positioned underneath the mattress 222 for monitoring the breathing state during sleep. Due to the respiratory chest wall motion, an oscillating signal is received. In the graph, four phases can be distinguished, when the person is lying on one side (1), on the back (2), on the other side (3) and on the belly (4), respectively. The large signal peaks in-between these phases occur, when the person moves from one position to the other. Clearly, for the device 100 being positioned underneath the mattress 222, sufficient signal amplitude can be obtained for monitoring the breathing state in the signal. For some applications, it may furthermore desirable to distinguish between the different breathing phases, e.g. inhaling, exhaling or holding breath. Due to interfering contributions of the user's body, the bed 200, the mattress 222 and the like, the assumption that moving away from the transceiving unit 110 relates to inhaling and moving towards it to exhaling does not hold for most measurement situations. Therefore, the shape of the signal itself may be used in order to analyze the breathing state according to the invention. For instance, a breathing pattern flattens for a short while after exhaling. This "flat" period can be used as a characteristic to discriminate inhaling from exhaling. Additionally or alternatively, a time ratio may be used as criterion, i.e. that inhaling involving muscle contraction takes shorter than exhaling involving muscle relaxation with a time ratio of 1:2, for example. These or similar criterions may be used to determine the different breathing phases.

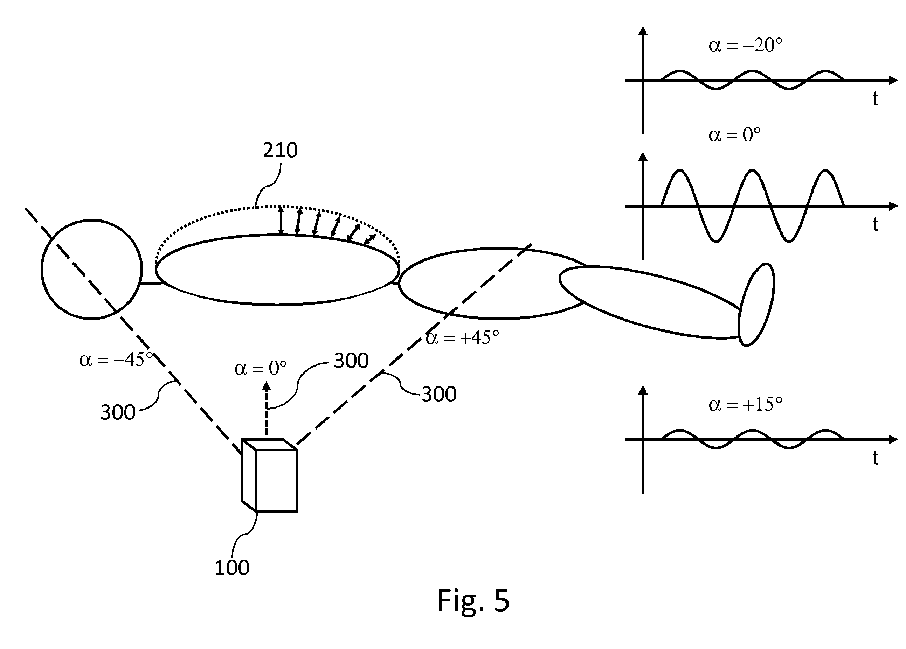

When using the device 100 to monitor vital signs of the user, the device 100 needs to adjust its direction of sensitivity 300 towards an area of interest 210 of the user's body corresponding to the kind of vital signs to be monitored. In the example for monitoring the breathing state, the area of interest 210 corresponds to the thorax region and some area around, typically an area of 25x25 cm. Fig. 5 illustrates the situation for monitoring vital signs during sleep. The area of interest 210 can be covered, if the direction of sensitivity 300 of the device 100 can be swayed within α=-45° and α=+45°, when the transceiving unit 110 has a reasonable distance from the person. As indicated in the graphs on the right in Fig. 5, the signal amplitude of the reflected signals varies strongly with the angle of detection (sensitivity angle α). In the example given, the strongest received signal comes from the central thorax area (e.g. α=0°). Thus, by carefully angle the device 100 for monitoring vital signs towards the respective area of interest 210, signal quality can be increased and even small signals can be detected. However, since the location of the user can vary in the bed 200, the position of the area of interest 210 is generally not constant. Thus, when the person is moving during his sleep, the person's chest could get out of the limited sensitivity area of the device 100 that is determined by its direction of sensitivity 300 (emission/receiving direction). In order to overcome this problem, the device 100 according to an embodiment of the present invention can vary its direction of sensitivity 300 within a predetermined range of possible sensitivity directions, e.g. from α=-45° to α=+45°, so that the direction of sensitivity 300 can be adjusted to the direction, in which the area of interest 210 is located, without re-arranging the device 100. Thus, the direction of sensitivity 300 can be adjusted within the predetermined range of possible sensitivity directions, the range being limited due to the hardware setup of the device 100.

Thus, first, the direction of sensitivity 300 is swayed for scanning the room for a signal by varying the transceiving or receiving direction of the device 100 to several or all possible directions in the predetermined range of possible sensitivity directions. For instance, when a device 100 comprises several antennas 111 or transceiving units 110, as described above, the antennas 111 (or transceiving units 110) can be successively selected for swaying the direction of sensitivity 300 of the device 100. Alternatively, the transceiving unit 110 may comprise a phased array, so that the direction of sensitivity 300 can be varied by beam forming (this will be described below). In a next step, when a direction of a best or strongest signal has been determined and thus the direction of the area of interest 210, the device 100 adjusts the direction of sensitivity 300 corresponding to this direction and collects the desired signals. When the person moves, the signal deteriorates or is lost. If this is determined, the device 100 restarts scanning the room in order to relocate the area of interest 210. In a preferred embodiment, the device 100 does not scan the full range, but starts with a progressive scan of the directions close to the last observed location of the area of interest 210 in order to recover the signal efficiently and fast. Consequently, the direction of sensitivity 300 of the device 100 according to one of the embodiments of the present invention can be controlled such, that it automatically follows the moving person.

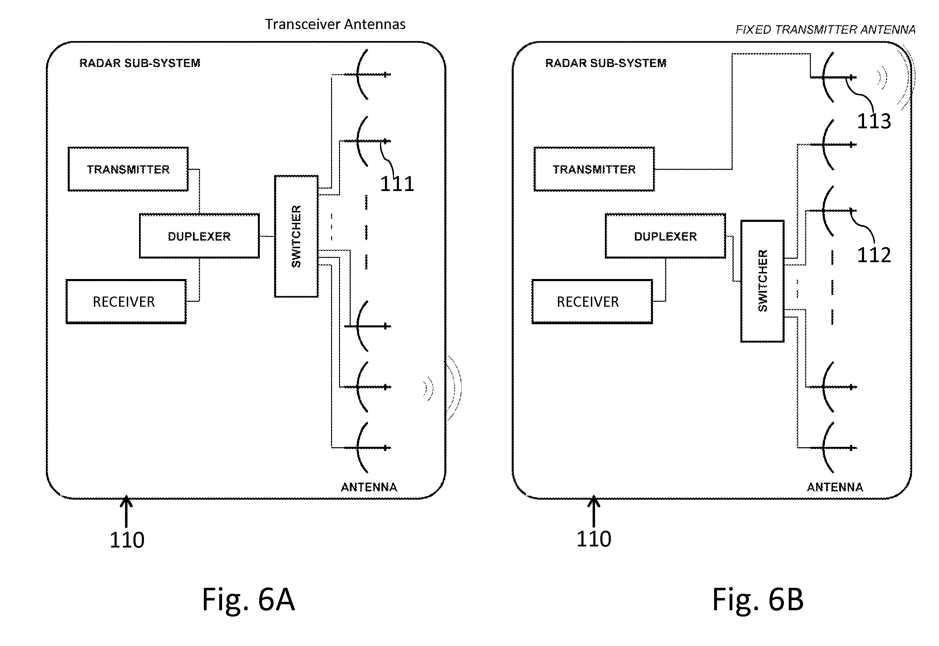

An embodiment for a radar sub-system having a plurality of transceiver antennas 111 is shown in Fig. 6A. A transmitter and a receiver are connected to a duplexer, which is connected to a switcher. The switcher is connected to the plurality of transceiver antennas 111 such that a radar beam is emitted in a predetermined direction. The switching of the radar sub-system is most easily multiplexed to the array of antennas 111 in the way that is commonly known e.g. for displays. For the intended compact size, the radar emitting device 100 can operate in a high GHz domain, at least K-band or higher.

According to another embodiment of a radar sub-system, the switching process can be made easier by splitting the antennas in transmitter and receiver antennas. For instance, as shown in Fig. 6B, one fixed transmitter antenna 113 can be used that is able to emit with a large angle of beam. Thus, the transmitter antenna 113 can cover a predetermined area, in which the area of interest 210 may be located. Furthermore, a plurality of directionally sensitive receiving antennas 112 is connected to a switcher. Hence, in this embodiment, the direction of sensitivity 300 of the device 100 can be adjusted by switching the receiving direction to the corresponding receiving antenna 112. To make the switching of the antennas 111 to the receiver possible and practical, it is not required that the radar gives absolute distance readings. It is sufficient to detect relative variations on a signal.

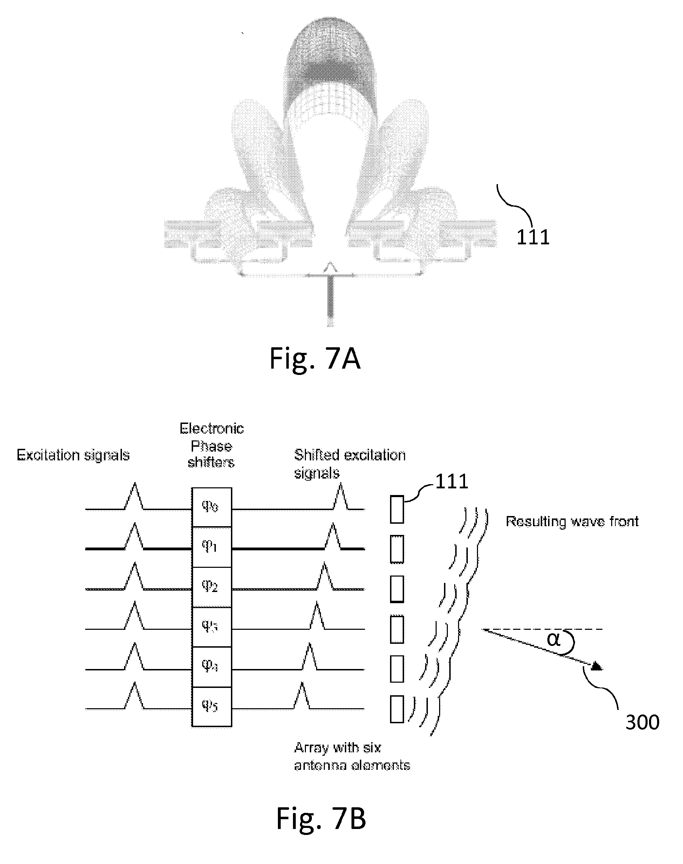

In a further embodiment of the present invention, the transceiving unit 110 of the device 100 comprises a phased antenna array capable of beam forming for directional signal transmission and/or reception, similar to the beam forming commonly known for multi-element ultrasound transducers. The spatial selectivity of a phased antenna array is achieved by taking advantage of constructive and destructive interferences in the wave front. Thus, the phase and relative amplitudes of the signals of each antenna 111 are controlled in order to change the direction of sensitivity 300 of the array. Usually, the antenna design determines the direction of sensitivity 300. The emission profile typically comprises a main lobe in the direction of high sensitivity and a few small side lobes. In Fig. 7A, the radiation characteristic of an array comprising four patch antenna elements 111 is shown. Several smaller side lobes exist next to the main lobe, but most of the energy is concentrated in the main lobe. Hence, in an array configuration, it is possible to have a rather focused main lobe. Furthermore, with a phased array of patch antennas 111, it is possible to sway the main lobe in different directions by introducing adjustable phase delays in feeding lines of the patch antennas 111. In Fig. 7B, a phased antenna array with six patch antenna elements 111 is shown. The excitation signals are supplied to electronic phase shifters, which shift the phases of the excitation signals according to the desired direction of the emitting beam. This results in a wave front emitted in a predetermined angle α (see arrow in fig. 7). Thus, by introducing different phase shifts in the lines feeding the antennas 111, the direction of the main lobe can be varied. Using different phase shifts while receiving signals with such an antenna array results in the antenna array being sensitive in specific directions, while suppressing incoming signals from other directions. So, if electronically adjustable phase shifters are used, it is possible to scan a given area for incoming signals and to actually adjust the direction of sensitivity 300 of the device 100 towards the area of interest 210, without having to move the antenna array mechanically and without having to provide directional antennas 111 for each direction.

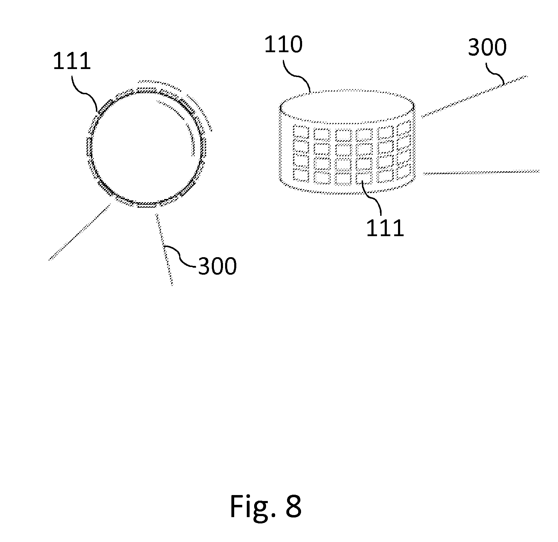

Instead of arranging the antenna elements 111 of a phased antenna array in a plane, the antenna elements 111 can also be arranged in other geometries. For instance, when the antenna elements 111 of a phased array are arranged on a cylindrical surface as shown in Fig. 8, the direction of sensitivity 300 can be rotated like the light cone of a lighting house by activating or combining appropriate antenna elements 111 and introducing variable delays or using adaptive filters for feeding the antenna elements 111. When the antenna elements 111 are not only segmented vertically but also horizontally (on right in Fig. 8), the emitted beam can also be horizontally bundled. Similarly, the antenna elements 111 may be arranged on a spherical surface. By these means, a complete room can be scanned for signals.

In general, the device 100 for monitoring vital signs according to one of the described embodiments may comprise an output unit for providing information, data or instructions to the user or health personnel. Likewise, input means may be provided e.g. for adjusting processing parameters for determining the direction of the area of interest 210, time intervals, angles and the like. Preferably, also a memory is provided to store at least one of the parameters, settings and data. In addition, applying a waveform to the same signal may make it easier to distinguish the primary signal from secondary reflections or other sources or radiation. In this case, the device 100 may comprise a modulation unit for frequency and/or amplitude modulation.

It is not required that the device 100 comprises all of these components. In a further aspect, the present invention may also be configured as a system. Thus, if the device 100 comprises a communication unit for wireless or wired data transfer with other external devices, some of these components may be provided separately. Optimally, the device 100 comprises the transceiving unit 110, the control unit 120 and a communication unit, so that the device 100 can be designed small and compact. Moreover, the device 100 or the system according to the present invention may be integrated in any system, e.g. in systems for patient monitoring at home or in general hospital wards, in safety systems for awareness monitoring in airplanes, trains or vehicles or in lifestyle systems for relaxing, and the like.

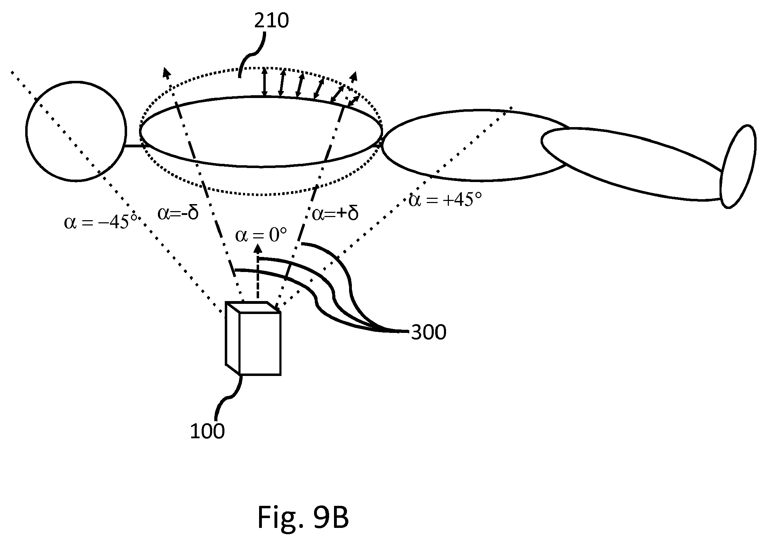

In a method for controlling a device for monitoring vital signs according to the present invention, the transceiving unit 110 is operated such that the direction of sensitivity 300 automatically follows the moving person. In a first step S100, the emitted beam of the device 100 is controlled, so that the direction of sensitivity 300 is swayed across a predetermined range between ±Δ, wherein ±Δ can be the full range the device 100 can detect. Preferably, the predetermined ranged is adjusted such that an area is scanned, in which the user may be located. In a second step S200, a direction αₘₐₓ is determined, from which the strongest or best signal was received in the first step (S100), and the angle α of the direction of sensitivity 300 is set to this direction αₘₐₓ. The signal quality may be determined using signal variance, signal amplitude, the signal-to-noise ration or the like. Referring to the example illustrated in Fig. 5 and 9B, if the device 100 sways its direction of sensitivity 300 between -45° and +45°, there will be a maximum at α = 0°. Measuring in this direction will result in a better signal-to-noise ratio, improving the measurement quality. In an optional further step (S300), it may be checked whether the person or the area of interest 210 (chest) has moved out of the direction of sensitivity 300. This can be performed in predetermined time intervals by swaying the sensitivity angle α of the device 100 in a small range α±δ (S310). Alternatively, it may be determined that the area of interest 210 is no longer aligned with the direction of sensitivity 300, when no more vital signs can be extracted from the received signals or when the received signals have changed significantly. In order to correct the direction of sensitivity 300 to a new direction of the area of interest 210, the sensitivity angle α is controlled such, that it sways and scans a small area around the last direction, that was used, in order to find the new best direction with the strongest signal. By these means, the direction of sensitivity 300 automatically follows, if the person moves.

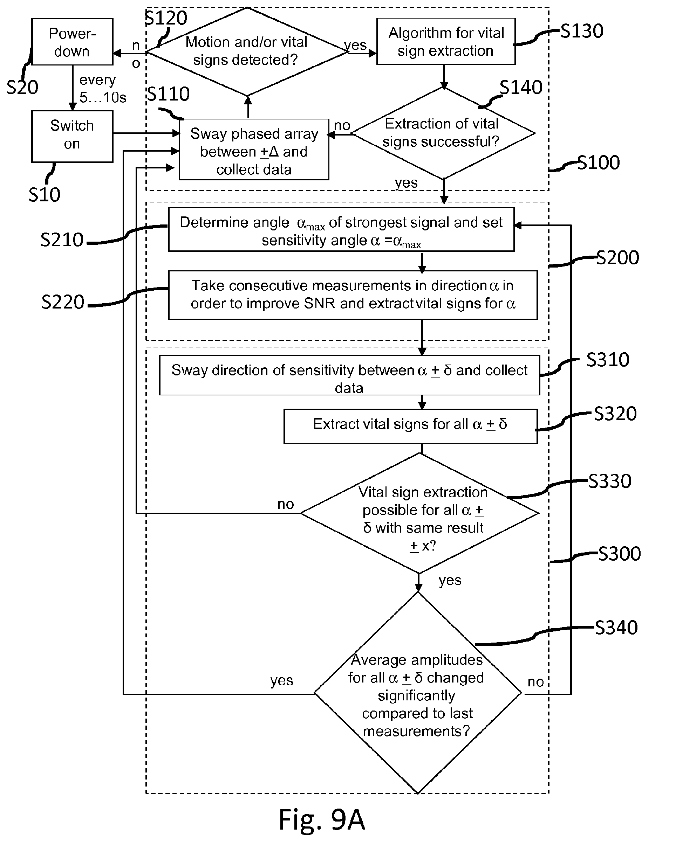

According to another more detailed embodiment of the method of the present invention as shown in Fig. 9A, first, when the device 100 is switched on, the direction of sensitivity 300, e.g. the main lobe of a phased antenna array, is swayed between a certain angular range ±Δ of possible directions and the reflected signals are recorded (S110). Next, an algorithm for vital sign extraction is applied to the recorded signals (S130) and it is determined whether vital signs can be extracted from the recorded signals (S 140). If this is not the case, the direction of sensitivity 300 is swayed again over the total range between ±Δ and data is collected (S110). Then, steps S110, S130 and S140 are repeated. If the extraction of vital signs was successful (S 140), the angle αₘₐₓ indicating the direction of the strongest signal is determined (S210), e.g. based on the signal amplitude, signal variance, signal-to-noise-ratio, or the like. Then, after setting the primary direction of sensitivity 300 to the direction of αₘₐₓ, signals are recorded for a predetermined time and vital signs are extracted from the recorded signals (S310). Possibly, the extracted vital signs are displayed or output by an output unit. In a preferred embodiment, the antenna array may sway its direction of sensitivity 300 (the sensitivity angle α) in a small range between α±δ and collect data (S310) either in predetermined time intervals, continuously or when certain conditions are met. These conditions may correspond to determining that the signal is lost or has changed significantly or that no vital signs can be extracted any longer. Then, vital signs are extracted for all sensitivity angles α±δ (S320) and it is determined whether the extraction of vital signs is possible for all sensitivity angles α±δ with approximately the same result within a certain threshold ±x (S330). If vital signs could not be extracted for all sensitivity angles α±δ, the routine re-starts with step S110 in order to update the primary direction of sensitivity 300. However, if vital signs could be extracted for all sensitivity angles α±δ, the amplitudes of signals received for all sensitivity angles may optionally be averaged and compared to average amplitudes of a previous measurement sequence (S340). If the average amplitudes have changed significantly, the direction of sensitivity 300 is possibly no longer well aligned with the area of interest 210 and the routine re-starts with step S 110 in order to relocate the area of interest 210. If the average amplitudes have remained approximately of the same order as the amplitudes of the last measurement sequences, the routine continues with determining the direction αₘₐₓ of the strongest signal (S220). This will result in a kind of fine-tuning for the determination of the direction αₘₐₓ of the strongest signal within a small range of sensitivity angles α±δ. However, it is also possible that the routine continues with step S220 to take consecutive measurements in direction of sensitivity angle α, if no significant change of the average amplitudes was determined (S340).

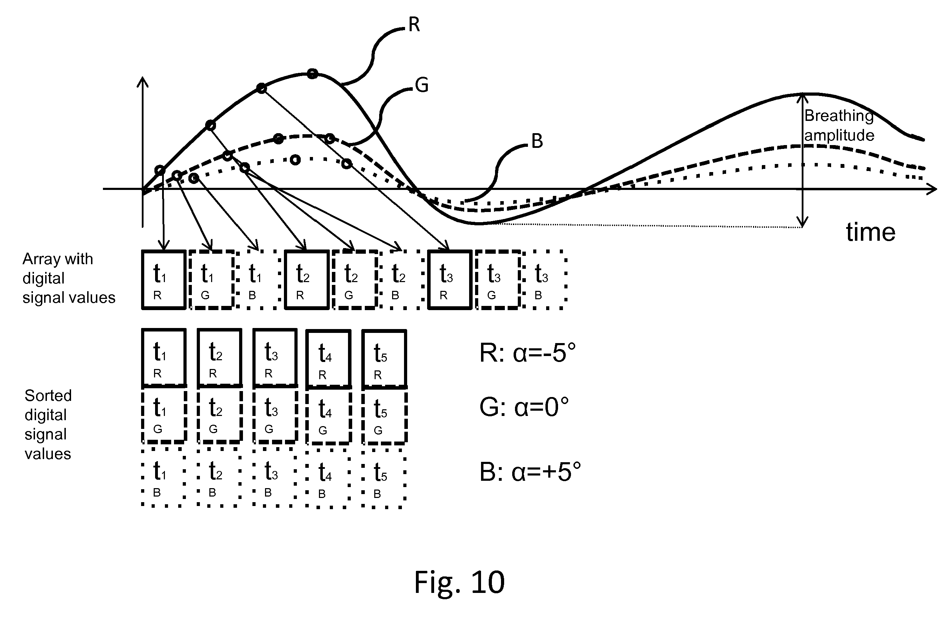

The step of swaying the direction of sensitivity 300 between α±δ (S310) is exemplified in Fig. 9B. The direction of sensitivity 300 can be adjusted successively to α-δ, α and α+δ, wherein α±δ lies within the range of possible directions (here α=±45°) predetermined by the hardware of the device 100. Measuring vital signs while swaying the direction of sensitivity 300 will result in signals, e.g. as shown in Fig. 10, wherein the direction of sensitivity 300 is continuously adjusted to α=-5°, α=0° and α=+5°.

In further modifications of the method as illustrated in Fig. 9A, one or more steps may be omitted, e.g. the method may only comprise either S320 and S330 or S340. Moreover, the step of swaying the direction of sensitivity 300 between α+6 (S310) may be omitted by setting δ=0°.

In addition, the method may comprise steps for automatically switching the device 100 on and off in a stand-by mode. For this, the device 100 may be activated in predetermined time intervals, e.g. every 10 seconds. When the device is switched on (S10), step S110 is started, wherein the direction of sensitivity 300 of the antenna array is swayed in the range of possible directions between ±Δ and signals are collected. Then, the device 100 is used as a detector of motion. If motion is detected (S 120), the algorithm for vital sign extraction is applied (S130) and the method continues according to one of the embodiments described above. If no motion was detected in S120, the device 100 is switched off again (S20) and the device 100 remains in the stand-by mode, wherein it is switched on from time to time (S10).

In Fig. 10, signal curves R, G and B are shown that are received for different sensitivity angles α, for instance R for α=-5°, G for α=0° and B at α=+5°. In this example, signals are recorded, while swaying the primary direction of sensitivity 300 of the device 100 continuously between α=-5° and α=+5° (S310). Thus, the sensitivity angle α is set to a first angle, e.g. +5° and a measurement is taken. Then, the sensitivity angle α is set to the next angle, e.g. 0°, and a measurement is taken from that next angle. This way, after all desired angles have been sampled, the measurements are re-started from the first angle. The resulting signal values are indicated in the first line below the graph in chronological order t_{1R}, t_{1G}, t_{1B} t_{2R}, t_{2G}, t_{2B}, t_{3R}, t_{3G}, t_{3B} etc. Since setting the sensitivity angle α of a phased antenna array with electronic phase shifters and also measuring at a specific angle α is extremely fast, it is possible to get virtually simultaneous measurement values from all possible different directions. Even when scanning the whole possible range ±Δ of directions of sensitivity 300, the measurements can be completed very fast, so that also the determination of the direction αₘₐₓ of the strongest signal (S210) takes only a few seconds.

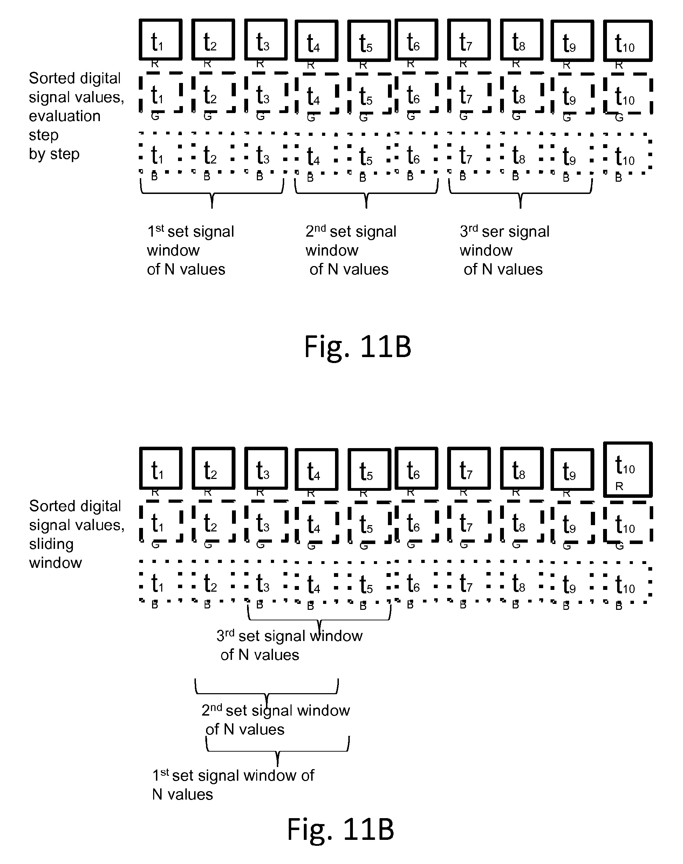

The recorded signals can be sorted with respect to the corresponding measurement angle and time, as shown in the lower part of Fig. 10. The processing of the signals can be then performed, e.g. block-wise or in a sliding-window manner. The two options are outlined in Fig. 11. In Fig. 11A, a block-wise evaluation of the sorted signal values is illustrated. The size of the signal windows can be set appropriately so that each window comprises the same number of values N. The signal values are processed step-by-step for each signal window. In Fig. 11B, the evaluation in a sliding-window manner is illustrated. Here, each window also comprises a predetermined number of values N, but subsequent windows are only shifted from each other by one signal value. Thus, in the sliding-window processing, subsequent windows comprise a subset of identical signal values. Using the sliding-window processing has the advantage that once the window is filled, it can be updated quickly by just adding the latest signal value and removing the oldest signal value, e.g. the transition from the first signal window to the second signal window (see Fig.11B). The block-wise processing, in contrast, requires waiting for a complete block to be filled with N sample data values.

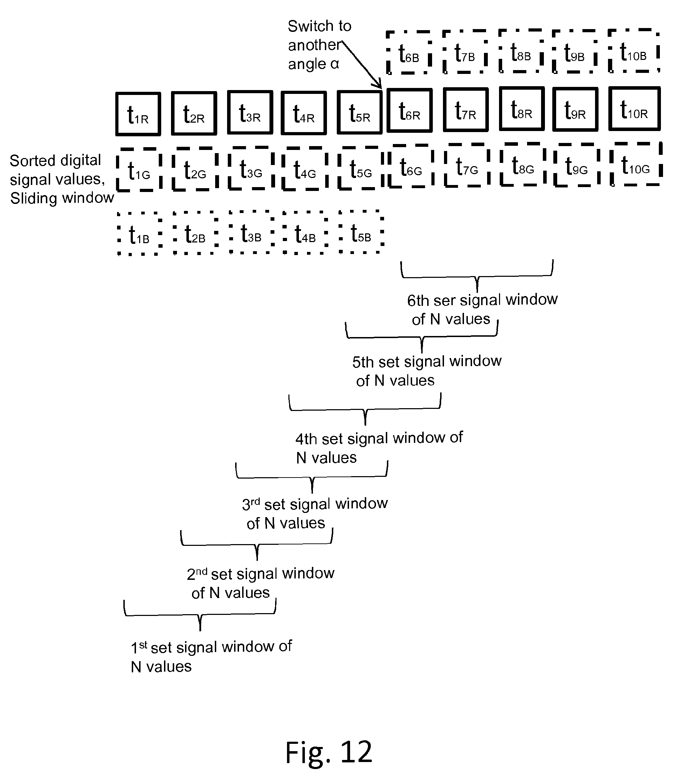

Fig. 12 illustrates an exemplary situation, when a new sensitivity angle αₘₐₓ has been determined by the signal processing to be the next optimal direction of sensitivity 300. As can be seen, the data collection in the former direction α=+5° is stopped after the fifth value t_{5B} and continued in a new direction. Due to the direction change, the 4th and 5th set of signals are possibly not usable and have to be dropped. It is also possible that all of the monitored angles are changed.

According to the present invention, vital signs of a user can be monitored by measuring electromagnetic signals reflected from a target area on the user's body (area of interest 210). Movements of the user and thus of the target area are automatically followed by scanning a large area, locating the target area and adjusting the measurement direction, i.e. the direction of sensitivity 300 correspondingly. Thus, vital signs can be monitored continuously and unobtrusively without re-arranging the device 100, without having to provide duplicate transceiving units 110 to cover the full area, in which the target area may be located, or having to instruct the user to aim the device 100 carefully or to keep the body inside a limited area. Therefore, the device, the system and the method of the present invention are very suitable for lifestyle applications, e.g. in paced-breathing products, devices aiding in relaxation or sleep, wake-up lights or alarm clocks. Moreover, if applied in general hospital wards or in home health care applications, an alarm may be triggered, when abnormalities in the vital signs are detected, e.g. in the monitored breathing state, heart rate, pulse form or the like. It is furthermore imaginable to apply the present invention to applications for pilot and/or passenger observation or for awareness monitoring in vehicles, trains, boats and airplanes in order to determine, for example, a heart attack or drowsiness and to take appropriate countermeasures.

## Claims

1. A device (100) for measuring vital signs, comprising:
a transceiving unit (110) capable of emitting electromagnetic signals and of directionally sensing reflected electromagnetic signals; and
a control unit (120) capable of determining a direction of interest (210) based on the sensed reflected signals and of adjusting a direction of sensitivity (300) of the transceiving unit (110) to the direction of interest (210).

2. The device (100) according to claim 1, wherein the electromagnetic signals are radar signals.

3. The device (100) according to claim 1 or 2, wherein the device (100) is capable of scanning an emission direction of a main beam of electromagnetic signals over a predetermined range of angles and/or by scanning the primary receiving direction over a predetermined range of angles.

4. The device (100) according to any one of the preceding claims, wherein the transceiving unit (110) comprises at least one emitting antenna (113) capable of emitting electromagnetic waves covering a predetermined area and a plurality of directionally sensitive receiver antennas (1112).

5. The device (100) according to any one of the preceding claims, wherein the transceiving unit (110) comprises at least one of a phased antenna array, a Yagi antenna, a parabolic antenna, a logarithmic-periodic antenna, a parabolic antenna, a corner-reflector antenna and a patch antenna.

6. The device (100) according to any one of the preceding claims, wherein the transceiving unit (110) comprises at least two transceivers (111), the transceiver (111) receiving the strongest reflected signals being selectable.

7. The device (100) according to any one of the preceding claims, wherein the transceiving unit (110) comprises at least two antennas (110) that are shifted in height and/or in phase and/or that have different wavelengths.

8. The device (100) according to any one of the preceding claims, wherein the control unit (120) is capable of determining the direction of interest (210) based on a signal-to-noise ratio, a signal amplitude and/or a signal variance of the sensed reflected signals.

9. The device (100) according to any one of the preceding claims, wherein the device (100) is capable of determining the direction of interest (210) in periodic intervals, at predetermined points in time and/or each time, when a predetermined condition is fulfilled.

10. The device (100) according to claim 9, wherein the predetermined condition is at least one of being switched on, receiving an input signal, detecting the presence of a person, detecting a significant change in the reflected signals amplitude, determining that no vital signs are obtainable from the sensed reflected signals and determining that the reflected signal has been lost.

11. The device (100) according to any one of the preceding claims, wherein the device (100) is operable in a stand-by mode, in which the device (100) determines in predetermined intervals, whether a person is present, and wherein the device (100) is activated for measuring vital signs, if a person is present.

12. The device (100) according to any one of the preceding claims used for determining a breathing state based on the shape of the reflected signals.

13. The device (100) according to any one of the preceding claims, comprising further a modulation unit for modulating an emitted signal and/or an input unit for receiving user input and/or a communication unit for data transmission to external devices and/or an output unit for visually and/or acoustically providing feedback, information and/or instructions.

14. A system for measuring vital signs, comprising:
a transceiving unit (110) capable of emitting electromagnetic signals and of directionally sensing reflected electromagnetic signals; and
a control unit (120) capable of determining a direction of interest (210) based on the sensed reflected signals and of adjusting a direction of sensitivity (300) of the transceiving unit (110) to the direction of interest (210);
wherein the transceiving unit (110) and the control unit (120) are adapted to be interconnected for wired and/or wireless communication.

15. A method for measuring vital signs, comprising:
emitting (S 110) electromagnetic signals;
directional sensing (S 110) of reflected signals;
determining (S210) a direction of interest (210) based on the sensed reflected signals;
adjusting (S210) a direction of sensitivity (300) to the direction of interest (210); and
receiving (S220) reflected signals from the direction of sensitivity and deriving vital signs from the reflected signals.
